# EUROPÄISCHE PATENTSCHRIFT

(11) **EP 1 133 496 B1**
(45) Veröffentlichungstag und Bekanntmachung des Hinweises auf die Patenterteilung: **21.04.2004**
(21) Anmeldenummer: 99958040.0
(22) Anmeldetag: 15.11.1999
(51) Int. Cl.: C07D 471/04, A01N 43/90, A61K 31/47

(54) **KRISTALLMODIFIKATION A VON 8-CYAN-1- CYCLOPROPYL-7- (1S,6S-2,8 -DIAZABICYCLO - 4.3.0]NONAN -8-YL)-6- FLUOR-1,4- DIHYDRO-4- OXO-3- CHINOLINCARBONSÄURE**
CRYSTAL MODIFICATION A OF 8-CYANO-1- CYCLOPROPYL -7-(1S,6S-2, 8-DIAZABICYCLO 4.3.0]NONAN -8-YL)-6- FLUORO-1, 4-DIHYDRO- 4-OXO-3- QUINOLINE CARBOXYLIC ACID
MODIFICATION CRISTALLINE A D'ACIDE 8-CYAN-1- CYCLOPROPYL -7-(1S,6S -2,8-DIAZABICYCLO 4.3.0]NONAN -8-YL)-6- FLUOR-1,4- DIHYDRO-4-OXO -3-CHINOLINCARBOXYLIQUE

(30) Priorität: 25.11.1998 DE 19854356
(43) Veröffentlichungstag der Anmeldung: 19.09.2001
(73) Patentinhaber: Bayer HealthCare AG, 51368 Leverkusen (DE)
(72) Erfinder: HIMMLER, Thomas, D-51519 Odenthal (DE); HALLENBACH, Werner, D-40789 Monheim (DE); RAST, Hubert, D-51381 Leverkusen (DE)
(86) Internationale Anmeldenummer: PCT/EP1999/008775
(87) Internationale Veröffentlichungsnummer: WO 2000/031075

(56) Entgegenhaltungen:
- DE-A- 19 546 249
- DE-A- 19 633 805

## Beschreibung

Die vorliegende Erfindung betrifft eine definierte Kristallmodifikation von 8-Cyan-1-cyclopropyl-7-(1S,6S-2,8-diazabicyclo[4.3.0]nonan-8-yl)-6-fluor-1,4-dihydro-4-oxo-3-chinolincarbonsäure, Verfahren zu ihrer Herstellung und ihre Verwendung in pharmazeutischen Zubereitungen.

DE-A-195 46 249 betrifft ein Monohydrat des 1-Cyclopropyl-7-([S,S]-2,8-diazabiycyclo[4.3.0]non-8-yl)6-fluor-1,4-dihydro-8-methoxy-4-oxo-3-chinolincarbonsäure Hydrochlorid (CDCH), ein Verfahren zu dessen Herstellung und pharmazeutische Zubereitungen, die dieses Monohydrat als Wirkstoff enthalten.

8-Cyan-1-cyclopropyl-7-(1S,6S-2,8-diazabicyclo[4.3.0]nonan-8-yl)-6-fluor-1,4-dihydro-4-oxo-3-chinolincarbonsäure der Formel (I) soll im folgenden als CCDC bezeichnet werden.

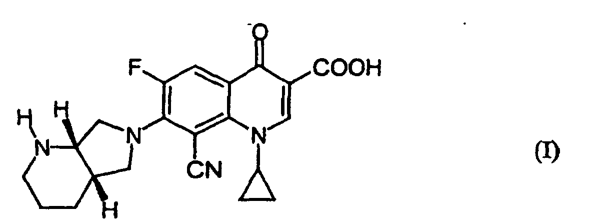

CCDC ist bekannt aus DE-A 19 633 805 oder PCT Amn-Nr. 97 903 260.4 Sie wird danach hergestellt durch Umsetzung von 7-Chlor-8-cyan-1-cyclopropyl-6-fluor-1,4-dihydro-4-oxo-3-chinolincarbonsäure mit (1S,6S)-2,8-Diazabicyclo[4.3.0]nonan in einem Gemisch aus Dimethylformamid und Acetonitril in Gegenwart einer Hilfsbase. Nach Versetzen mit Wasser wird CCDC mit Dichlormethan aus Wasser extrahiert und durch Entfernen des Extraktionsmittels isoliert. Man erhält dabei ein Pulver, das keine eindeutige Kristallmodifikation aufweist. Das Pulver ist vielmehr zu großen Teilen amorph und kann Gemische verschiedener Kristallmodifikationen enthalten. Sollte durch Zufall eine einheitliche Kristallmodifikation entstehen ist unklar, wie sie extrahiert und definiert erhalten werden kann. Für die Herstellung von Arzneimitteln ist jedoch Voraussetzung, daß für einen Wirkstoff, der in verschiedenen Kristallmodifikationen vorliegen kann, eindeutig angegeben wird, in welcher Kristallmodifikation er zur Herstellung des Mittels eingesetzt wird.

Das z.T. amorphe Pulver, das nach dem oben skizzierten Herstellverfahren erhalten wird, ist zudem hygroskopisch. Amorphe Feststoffe, und besonders hygroskopische Feststoffe, sind in der galenischen Verarbeitung schlecht handzuhaben, da sie beispielsweise geringe Schüttdichten und mangelhafte Fließeigenschaften aufweisen können. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehaltes oder der Stabilität in den produzierten Festformulierungen zu erhalten.

Der Erfindung liegt daher die Aufgabe zugrunde, eine kristalline Form definierter Modifikation von CCDC herzustellen, die aufgrund ihrer physikalischen Eigenschaften, insbesondere ihrer Kristalleigenschaften und ihres Verhaltens gegenüber Wasser in galenischen Formulierungen gut zu handhaben ist.

Diese Aufgabe wird erfindungsgemäß durch eine neue, kristalline Form von CCDC gelöst, die nachstehend als Modifikation A bezeichnet wird.

Gegenstand der Erfindung ist daher die kristalline Modifikation A von CCDC, die dadurch gekennzeichnet ist, daß sie ein Röntgen-Pulverdiffraktogramm mit den in der folgenden Tabelle 1 angegebene Reflexlagen (2 Theta) hoher und mittlerer Intensität (> 30% relative Intensität) aufweist.

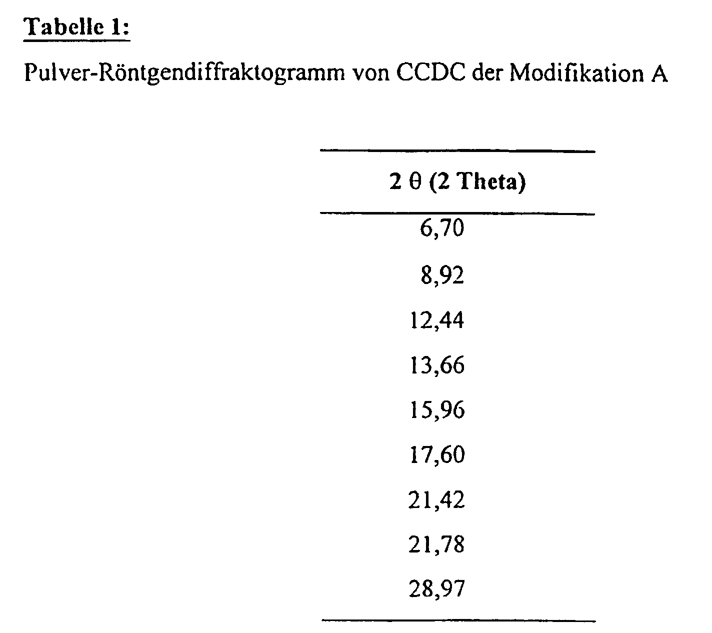

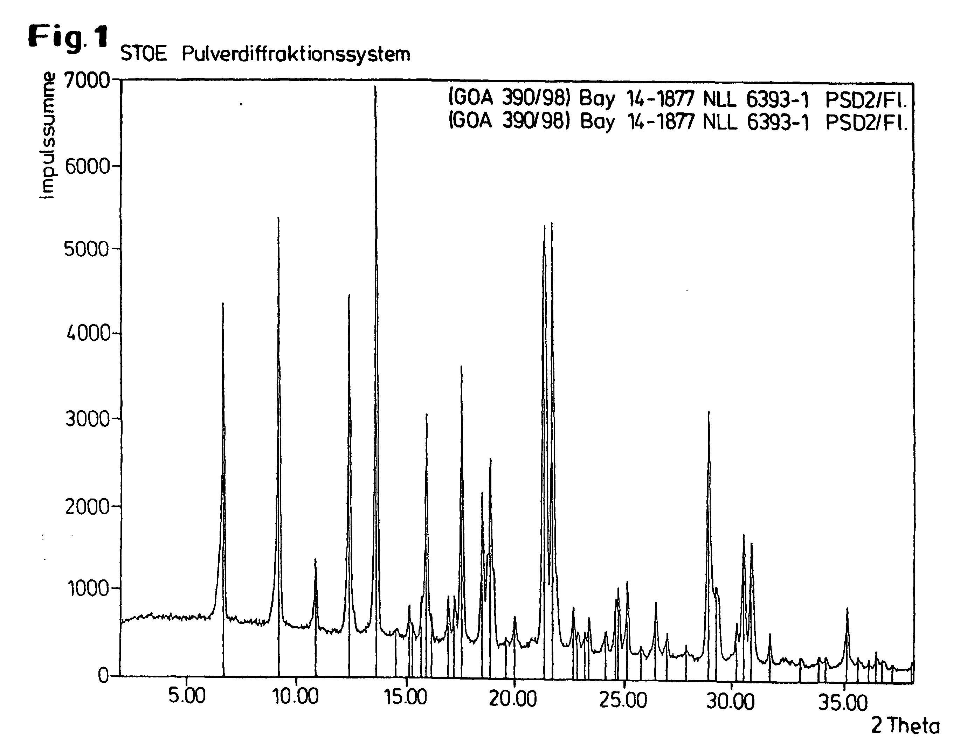

Ein charakteristisches Pulver-Röntgendiffraktogramm der Modifikation A ist auch in der Abbildung 1 wiedergegeben.

Die erfindungsgemäße Modifikation A von CCDC unterscheidet sich außerdem in einer Reihe weiterer Eigenschaften von den anderen Formen der CCDC. Diese Eigenschaften können einzeln oder gemeinsam mit den übrigen Parametern zur Charakterisierung der erfindungsgemäßen Modifikation A der CCDC dienen.

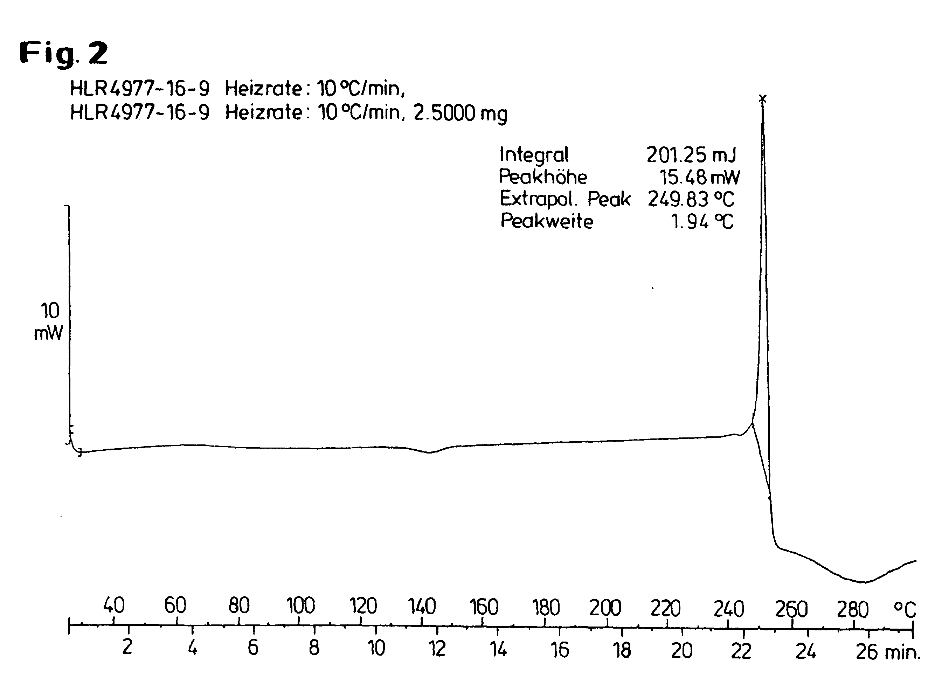

CCDC der Modifikation A ist u.a. dadurch gekennzeichnet, daß es einen mit Hilfe der Differentialthermoanalyse (DTA) bestimmten Schmelzpunkt von 249°C bis 252°C hat. Ein charakteristisches Differentialthermodiagramm ist in der Abbildung 2 wiedergegeben.

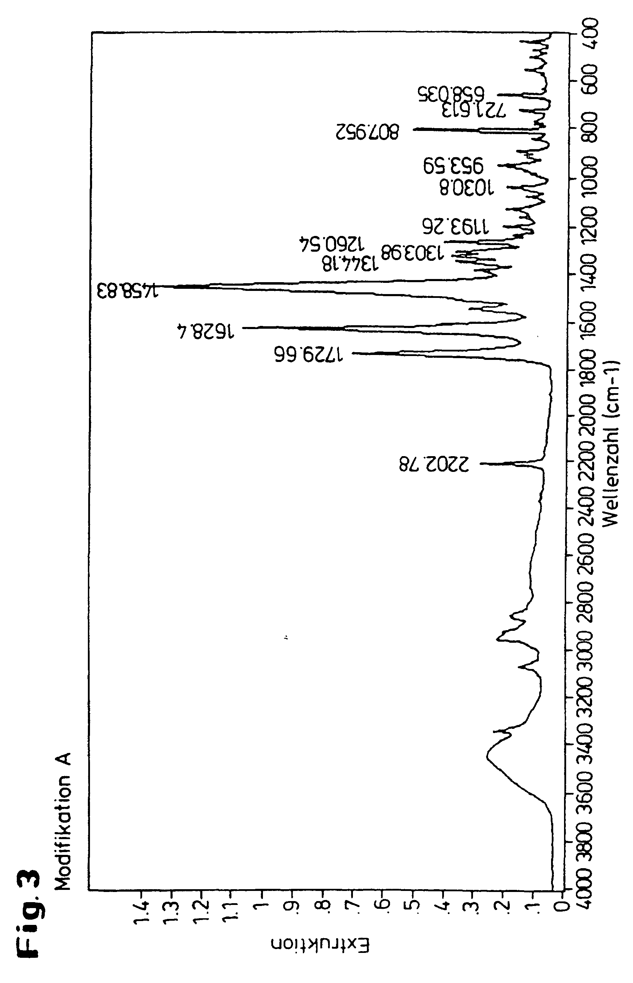

CCDC der Modifikation A ist weiterhin dadurch gekennzeichnet, daß es ein in KBr gemessenes Infrarotspektrum wie in Abbildung 3 gezeigt besitzt.

CCDC der Modifikation A ist weiterhin dadurch gekennzeichnet, daß es nach dem im folgenden angegebenen Herstellverfahren erhältlich ist. Die Kristallmodifikation A von CCDC wird dadurch erhalten, daß man CCDC unbekannter Modifikation oder amorphes CCDC in Wasser oder einem Alkohol-Wasser-Gemisch in der Wärme löst, anschließend einen Alkohol zugibt und nach Abkühlen auf Raumtemperatur den ausgefallenen Feststoff isoliert.

Als Alkohol werden in einer bevorzugten Ausführungsform Ethanol und Isopropanol verwendet.

CCDC der Kristallmodifikation A ist überraschend stabil und wandelt sich auch bei längerer Lagerung nicht in eine andere Kristallmodifikation oder die amorphe Form um. Darüber hinaus neigt die Modifikation A im Vergleich mit amorphem CCDC weit weniger zur Aufnahme von Wasser aus der Luft. Es ist aus diesen Gründen hervorragend zur Herstellung von Tabletten oder anderen Festformulierungen geeignet. Durch seine Stabilität verleiht es diesen Formulierungen die gewünschte lang andauernde Lagerstabilität. Mit der Kristallmodifikation A können damit definiert und gezielt stabile feste Zubereitungen von CCDC hergestellt werden.

CCDC der Kristallmodifikation A ist hervorragend gegen pathogene Bakterien auf dem Gebiet der Human- oder Tiermedizin wirksam. Sein breites Einsatzgebiet entspricht dem von CCDC.

Das Röntgen-Pulverdiffraktogramm zur Charakterisierung der Kristallmodifikation A von CCDC wurde mit einem Transmissions-Diffraktometer STADI-P mit ortsempfindlichem Detektor (PSD2) der Fa. Stoe erhalten.

Der Schmelzpunkt der Differentialthermoanalyse wurde mit dem Gerät DSC 820 der Fa. Mettler-Toledo erhalten. Dabei wurde die Probe von CCDC der Kristallmodifikation A in einem Aluminiumtiegel mit 10 K/min an der Luft aufgeheizt.

Das IR-Spektrum wurde mit dem Gerät FTS 60 A der Fa. Biorad in KBr erhalten.

Die folgenden Beispiele illustrieren die Erfindung ohne sie einzuschränken. Die in den folgenden Beispielen beschriebenen Verdünnungsmittel/Basensysteme sind besonders bevorzugt.

### Vergleichsbeispiel

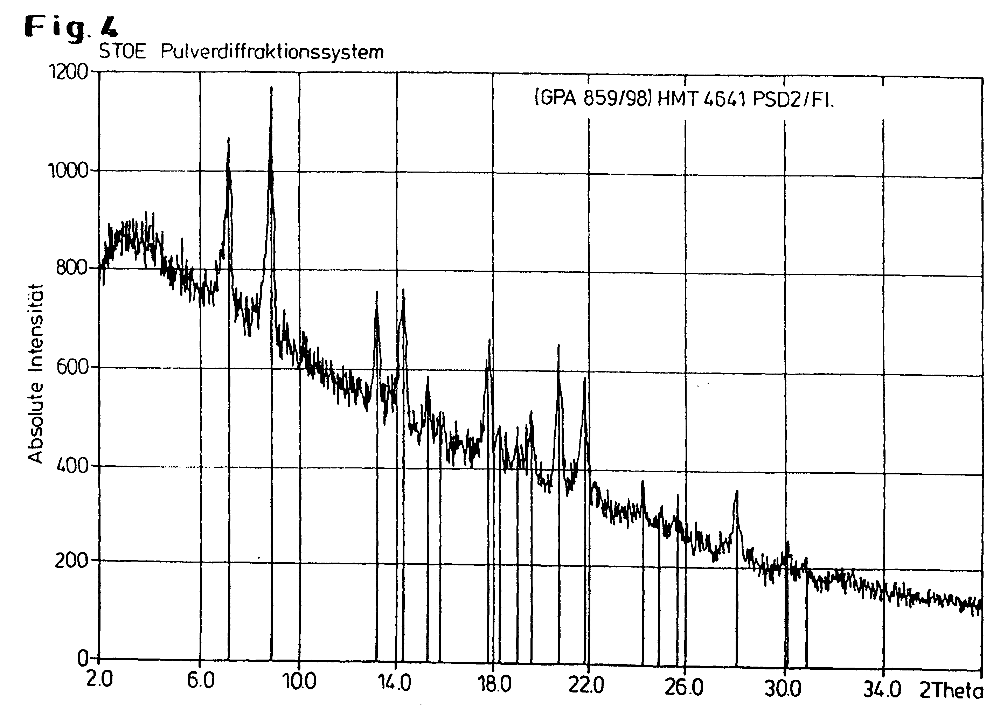

Eine Mischung aus 3,07 g 7-Chlor-8-cyan-1-cyclopropyl-6-fluor-1,4-dihydro-4-oxo-3-chinolincarbonsäure, 1,39 g (1S,6S)-2,8-Diazabicyclo[4.3.0]nonan, 2,24 g 1,4-Diazabicyclo[2.2.2]octan (DABCO), 29,5 ml Dimethylformamid und 29,5 ml Acetonitril wird 16 Stunden bei Raumtemperatur gerührt. Das Reaktionsgemisch wird bei 60°C Badtemperatur am Rotationsverdampfer eingeengt und der Rückstand in 10 ml Wasser aufgenommen. Die resultierende Lösung wird mit verdünnter Salzsäure auf pH 7 gestellt und der Feststoff abfiltriert. Das Filtrat wird dreimal mit je 20 ml Dichlormethan ausgeschüttelt. Man trocknet die organische Phase über Natriumsulfat, filtriert und engt das Filtrat am Rotationsverdampfer bei 60°C Badtemperatur ein. Man erhält 2,4 g hellbraunen Feststoff, der das in der Abbildung 4 gezeigte Röntgen-Pulverdiffraktogramm aufweist und demnach zum großen Teil amorph ist.

Der gemäß dieser Vorschrift erhaltene Feststoff nimmt bei einer relativen Luftfeuchtigkeit von 95 % (eingestellt durch eine gesättigte Lösung mit Bodensatz von Na₂HPO₄ x 12 H₂O in Wasser) innerhalb eines Tages ca. 17 Gewichtsprozent Wasser auf.

### Beispiel 1

617 g CCDC beliebiger Modifikation werden in 6170 ml Chloroform gelöst. Man gibt 100 g Natriumsulfat hinzu, rührt 5 Minuten und filtriert dann über 50 g Kieselgur, welches mit 100 ml Chloroform nachgewaschen wird. Das Lösungsmittel wird am Rotationsverdampfer bis zu einem Restdruck von 10 mbar abdestilliert, wodurch ein glasartiger Rückstand resultiert. Zu diesem Rückstand gibt man 740 ml Wasser und 740 ml Ethanol und erwärmt auf 60°C bis alles in Lösung gegangen ist. Diese Lösung wird dann in 17 Liter siedendes Ethanol gegeben. Man läßt für 5 Minuten weiter Sieden und kühlt dann innerhalb einer Stunde auf 35°C ab. Die ausgefallenen Kristalle werden abgesaugt und etwa 16 Stunden bei 20°C und dann bei 30°C im Vakuum bis zur Gewichtskonstanz getrocknet.

Man erhält 530 g Feststoff, der das in der Abbildung 1 gezeigte Röntgen-Pulverdiffraktogramm, das in der Abbildung 2 gezeigte Differentialthermodiagramm und das in der Abbildung 3 gezeigte IR-Spektrum aufweist.

Der gemäß dieser Vorschrift erhaltene Feststoff nimmt bei einer relativen Luftfeuchtigkeit von 95 % (eingestellt durch eine gesättigte Lösung mit Bodensatz von Na₂HPO₄ x 12 H₂O in Wasser) innerhalb eines Tages ca. 3 Gewichtsprozent Wasser auf.

### Beispiel 2

2 g CCDC unbekannter Modifikation werden in 4 ml Wasser gelöst. Man gibt 4 ml Isopropanol hinzu, beginnt das Reaktionsgemisch unter Rühren langsam zu erwärmen und gibt dann weitere 32 ml Isopropanol hinzu. Die resultierende klare Lösung wird bis zum Sieden erhitzt. Dabei trübt sich die Lösung ein und scheidet innerhalb kurzer Zeit Kristalle ab. Nach drei Minuten Rückfluß wird die Heizung entfernt und der Ansatz ohne Rühren 3 bis 4 Stunden stehen gelassen. Dann wird abgesaugt und der Feststoff mit Isopropanol nachgewaschen. Man trocknet an der Luft bis zur Gewichtskonstanz. Es werden 1,54 g Feststoff erhalten, der ein zu dem in der Abbildung 1 gezeigtes identisches Röntgen-Pulverdiffraktogramm, ein zu dem in der Abbildung 2 gezeigten identisches Differentialthermodiagramm und ein zu dem in der Abbildung 3 gezeigten identisches IR-Spektrum aufweist.

## Patentansprüche

1. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation A, **dadurch gekennzeichnet, daß** sie ein Röntgen-Pulverdiffraktogramm mit folgenden Reflexlagen (2 Theta) hoher und mittlerer Intensität aufweist

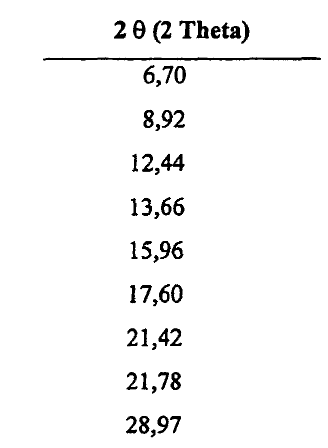

2. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation A, **dadurch gekennzeichnet, daß** sie ein Röntgen-Pulverdiffraktogramm mit folgenden Reflexlagen (2 Theta) hoher und mittlerer Intensität aufweist 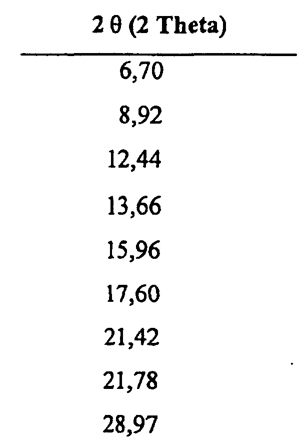 und einen durch DTA ermittelten Schmelzpunkt von 249°C bis 252°C besitzt.

3. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation A, dadurch erhältlich, daß CCDC unbekannter Modifikation oder amorphes CCDC in Wasser oder einem Wasser-Alkohol-Gemisch gelöst und nach Alkoholzugabe in der Wärme ausgefällt wird.

4. Verfahren zur Herstellung von CCDC der Modifikation A gemäß Anspruch 1, **dadurch gekennzeichnet, daß** CCDC unbekannter Modifikation oder amorphes CCDC in Wasser oder einem Wasser-Alkohol-Gemisch gelöst und in der Wärme nach Alkoholzugabe ausgefällt wird.

5. Verfahren zur Herstellung von CCDC der Modifikation A gemäß Anspruch 4, **dadurch gekennzeichnet, daß** dieser Alkohol Ethanol oder Isopropanol ist.

6. Arzneimittel, **dadurch gekennzeichnet, daß** es neben üblichen Hilfs- und Trägerstoffen CCDC der Modifikation A gemäß einem der Ansprüche 1 bis 3 enthält.

7. Verwendung von CCDC der Modifikation A gemäß einem der Ansprüche 1 bis 3 zur Herstellung von Arzneimitteln.

8. Verwendung von CCDC der Modifikation A gemäß einem der Ansprüche 1 bis 3 zur Herstellung von antibakteriellen Mitteln.

## Claims

1. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification A, **characterized in that** it has an X-ray powder diffractogram with the following reflection signals (2 theta) of high and medium intensity

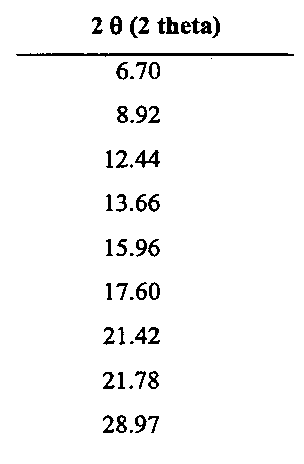

2. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification A, **characterized in that** it has an X-ray powder diffractogram with the following reflection signals (2 theta) of high and medium intensity 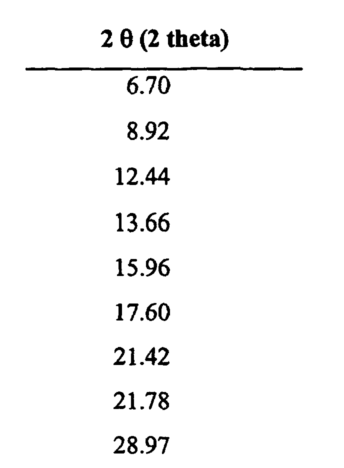 and a melting point, determined by DTA, of from 249°C to 252°C.

3. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification A, obtainable by dissolving CCDC of unknown modification or amorphous CCDC in water or a water/alcohol mixture, followed by hot precipitation after the addition of alcohol.

4. Process for preparing CCDC of the modification A according to Claim 1, **characterized in that** CCDC of unknown modification or amorphous CCDC is dissolved in water or a water/alcohol mixture and precipitated hot after the addition of alcohol.

5. Process for preparing CCDC of the modification A according to Claim 4, **characterized in that** the alcohol used is ethanol or isopropanol.

6. Medicament, **characterized in that** it comprises, in addition to customary auxiliaries and excipients, CCDC of the modification A according to any of Claims 1 to 3.

7. Use of CCDC of the modification A according to any of Claims 1 to 3 for preparing medicaments.

8. Use of CCDC of the modification A according to any of Claims 1 to 3 for preparing antibacterial compositions.

## Revendications

1. Acide 8-cyano-1-cyclopropyl-7-(1S,6S-2,8-diazabicyclo[4.3.0.]nonan-8-yl)-6-fluoro-1,4-dihydro-4-oxo-3-quinoléinecarboxylique (CCDC) de modification cristalline A, **caractérisé en ce qu'**il présente un diffractogramme aux rayons X pour substances pulvérulentes présentant les couches réflexes suivantes (2 thêta) d'intensité élevée et moyenne:

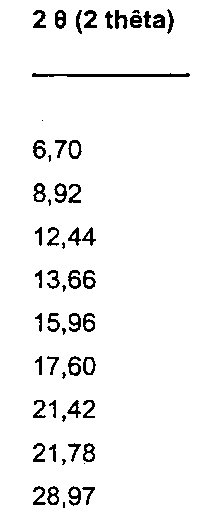

2. Acide 8-cyano-1-cyclopropyl-7-(1S,6S-2,8-diazabicyclo[4.3.0.]nonan-8-yl)-6-fluoro-1,4-dihydro-4-oxo-3-quinoléinecarboxylique (CCDC) de la modification cristalline A, **caractérisé en ce qu'**il présente un diffractogramme aux rayons X pour substances pulvérulentes présentant les couches réflexes suivantes (2 thêta) d'intensité élevée et moyenne: 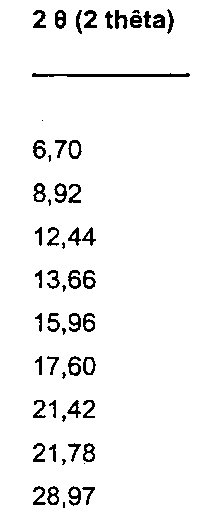 et possède un point de fusion, déterminé par DTA, de 249°C à 252°C.

3. Acide 8-cyano-1-cyclopropyl-7-(1S,6S-2,8-diazabicyclo[4.3.0.]nonan-8-yl)-6-fluoro-1,4-dihydro-4-oxo-3-quinoléinecarboxylique (CCDC) de modification cristalline A obtenu par le fait qu'un CCDC de modification inconnue ou un CCDC amorphe est dissous dans l'eau ou dans un mélange eau - alcool et est précipité à la chaleur après addition d'alcool.

4. Procédé de préparation de CCDC de modification A conformément à la revendication 1, **caractérisé en ce qu'**un CCDC de modification inconnue ou un CCDC amorphe est dissous dans l'eau ou dans un mélange eau - alcool et est précipité à la chaleur après addition d'alcool.

5. Procédé de préparation de CCDC de modification A conformément à la revendication 4, **caractérisé en ce que** cet alcool est l'éthanol ou l'isopropanol.

6. Médicament **caractérisé en ce qu'**outre les adjuvants et excipients courants, il contient un CCDC de modification A conformément à l'une des revendications 1 à 3.

7. Mise en oeuvre de CCDC de modification A selon l'une des revendications 1 à 3 pour la préparation de médicaments.

8. Mise en oeuvre de CCDC de modification A selon l'une des revendications 1 à 3 pour la préparation d'agents antibactériens.
